# EUROPEAN PATENT APPLICATION

(11) **EP 4 585 901 A1**
(43) Date of publication of application: **16.07.2025**
(21) Application number: 23862870.5
(22) Date of filing: 09.08.2023
(51) Int. Cl.: G01N 15/00, G01N 15/10, G01N 23/02, G01N 23/2206, G01N 23/223, G01N 1/02

(54) **ANALYSIS SYSTEM AND ANALYSIS METHOD**

(30) Priority: 08.09.2022 JP 2022143233
(71) Applicant: HORIBA, Ltd., Minami-ku Kyoto-shi Kyoto 601-8510 (JP)
(72) Inventor: MATSUMOTO, Erika, Kyoto-shi, Kyoto 601-8510 (JP); SANDO, Shota, Kyoto-shi, Kyoto 601-8510 (JP); HIROSE, Jun, Kyoto-shi, Kyoto 601-8510 (JP)
(74) Representative: Müller Hoffmann & Partner
(86) International application number: PCT/JP2023/029084
(87) International publication number: WO 2024/053328

(57) **Abstract**

To chronologically monitor the environment in a predetermined facility at a short period of time, an analysis system (100) includes a data acquisition unit (1) and an analysis unit (35). The data acquisition unit (1) collects particulate matter existing in the predetermined facility at a predetermined period, and calculates chronological data (RD) relating to the collected particulate matter. The analysis unit (35) acquires environment information relating to the environment in the predetermined facility, based on the data (RD) relating to the particulate matter calculated by the data acquisition unit (1). The data (RD) relating to the particulate matter includes element data relating to elements contained in the particulate matter.

## Description

### TECHNICAL FIELD

The present invention relates to an analysis system that performs analysis relating to the environment in a predetermined facility, and an analysis method relating to the environment in a predetermined facility.

### BACKGROUND ART

There is known a system that acquires data relating to particulate matter, such as information relating to elements contained in the particulate matter existing in the atmosphere or the like (e.g., elements contained in the particulate matter, contents of the elements), and performs analysis relating to the particulate matter based on the acquired data (e.g., see Patent Citation 1).

### PRIOR ART CITATIONS

### PATENT CITATION

Patent Citation 1: WO2018/117146

### SUMMARY OF INVENTION

### TECHNICAL PROBLEM

In recent years, along with higher accuracy and purity of quality of a product, there are requests to monitor the environment in a facility (such as a factory) in which the product is produced. In this case, conventionally, collection of objects to be analyzed and analysis thereof are performed in different sites. If the objects to be analyzed are inorganic substances, for example, collected liquid is left in the facility to be monitored for a few days, to collect the inorganic substances, and then the collected liquid is sent to a separate analysis institute, so that the inorganic substances are analyzed in the institute. In this way, conventionally, objects to be analyzed cannot be collected and analyzed at a short period of time, and it was difficult to chronologically monitor the environment in the facility.

It is an object of the present invention to chronologically monitor the environment in a predetermined facility at a short period of time.

### TECHNICAL SOLUTION

Hereinafter, a plurality of embodiments are described as means for solving the problem. These embodiments can be arbitrarily combined as necessary.

An analysis system according to an aspect of the present invention is a system that performs analysis relating to the environment in a predetermined facility. The analysis system includes a data acquisition unit and an analysis unit. The data acquisition unit collects particulate matter existing in the predetermined facility at a predetermined period, and calculates chronological data relating to the collected particulate matter. The analysis unit acquires environment information relating to the environment in the predetermined facility, based on the data relating to the particulate matter calculated by the data acquisition unit. The data relating to the particulate matter includes element data relating to elements contained in the particulate matter.

This time, analysis relating to the particulate matter existing in the predetermined facility whose environment was to be monitored was performed, and it was found that the environment in the facility can be monitored based on a result of the analysis. Based on this knowledge, the above analysis system collects the particulate matter existing in the predetermined facility at a short period of time, and calculates the data relating to the particulate matter at a short period of time, to calculate the chronological data relating to the particulate matter. **In** addition, based on the chronological data relating to the particulate matter, the environment information relating to the environment in the predetermined facility is acquired. **In** this way, the environment in the predetermined facility can be chronologically monitored at a short period of time. **In** addition, because the data relating to the particulate matter includes the element data relating to elements contained in the particulate matter, properties of the particulate matter existing in the predetermined facility can be predicted, for example, and hence the environment in the predetermined facility can be monitored in detail.

**In** the above analysis system, the data acquisition unit may collect the particulate matter at a plurality of places in the predetermined facility, to calculate the data relating to the particulate matter at each of the plurality of places. **In** this case, the above analysis system may further include an output unit. The output unit simultaneously outputs the data relating to the particulate matter at the plurality of places. **In** this way, chronological changes of the particulate matter at the plurality of places in the predetermined facility can be visually checked. **In** addition, based on the chronological changes of the particulate matter, changes of the environment in the predetermined facility can be chronologically monitored in more detail.

**In** the above analysis system, the environment information may include correlation between a plurality of the data relating to the particulate matter. **In** this way, for example, increase or decrease tendency or the like of the contents of the plurality of elements contained in the particulate matter can be acquired, and hence the environment in the predetermined facility can be monitored in detail.

**In** the above analysis system, the data acquisition unit may include a collection unit configured to collect particulate matter in the predetermined facility. **In** this case, the collection unit may have a plurality of ends extending to a plurality of places in the predetermined facility to collect the particulate matter from the plurality of places. In this way, the single data acquisition unit (collection unit) can collect the particulate matter at the plurality of places in the predetermined facility.

The above analysis system may further include an output unit configured to output a scatter diagram of the plurality of element data. In this way, a correlation relationship among the plurality of elements contained in the particulate matter can be visually checked.

In the above analysis system, the data relating to the particulate matter may include data relating to mass concentration of the particulate matter. In this way, information relating to quantity (concentration) of the particulate matter contained in the predetermined facility can be acquired. As a result, the environment in the predetermined facility can be monitored in detail.

The data acquisition unit may include a particle size classifier configured to classify the particulate matter by its particle size. In this way, data relating to the particulate matter having a specific particle size can be acquired. As a result, the environment in the predetermined facility can be monitored in detail.

An analysis method according to another aspect of the present invention is an analysis method for performing analysis relating to the environment in a predetermined facility. The analysis method includes the steps of:
collecting particulate matter existing in the predetermined facility at a predetermined period;
calculating chronological data relating to the collected particulate matter; and
acquiring environment information relating to the environment in the predetermined facility, based on the calculated data relating to the particulate matter.

In the above analysis method, the particulate matter existing in the predetermined facility is collected at a short period of time, and the data relating to the particulate matter is calculated at a short period of time, to calculate the chronological data relating to the particulate matter. In addition, the environment information relating to the environment in the predetermined facility is acquired based on the chronological data relating to the particulate matter. In this way, the environment in the predetermined facility can be chronologically monitored at a short period of time.

### ADVANTAGEOUS EFFECTS

By acquiring the chronological data relating to the particulate matter in the predetermined facility, the environment in the predetermined facility can be chronologically monitored at a short period of time.

### BRIEF DESCRIPTION OF DRAWINGS

Fig. 1 is a diagram illustrating a structure of an analysis system.
Fig. 2 is a diagram illustrating a structural example of an analysis device.
Fig. 3 is a diagram illustrating an example of a collection unit having a plurality of other ends.
Fig. 4 is a diagram illustrating a functional block structure of an analysis server.
Fig. 5 is a diagram illustrating an example of a simultaneous display of graphs of a plurality of data.
Fig. 6 is a flowchart illustrating a peak search operation.
Fig. 7 is a flowchart illustrating an automatic correlation calculation operation.
Fig. 8 is a diagram illustrating an example of a scatter diagram in a case where there are a plurality of correlations between two data.
Fig. 9 is a diagram illustrating an example when displaying only a scatter diagram having a large correlation.
Fig. 10 is a diagram illustrating an example when the scatter diagram having a large correlation is displayed in highlight.
Fig. 11 is a diagram illustrating an example of a state where chronological changes of two data values having a large correlation are displayed in graphs.
Fig. 12 is a diagram illustrating an example of a data graph display.

### DESCRIPTION OF EMBODIMENTS

### 1. First Embodiment

### (1) Analysis System

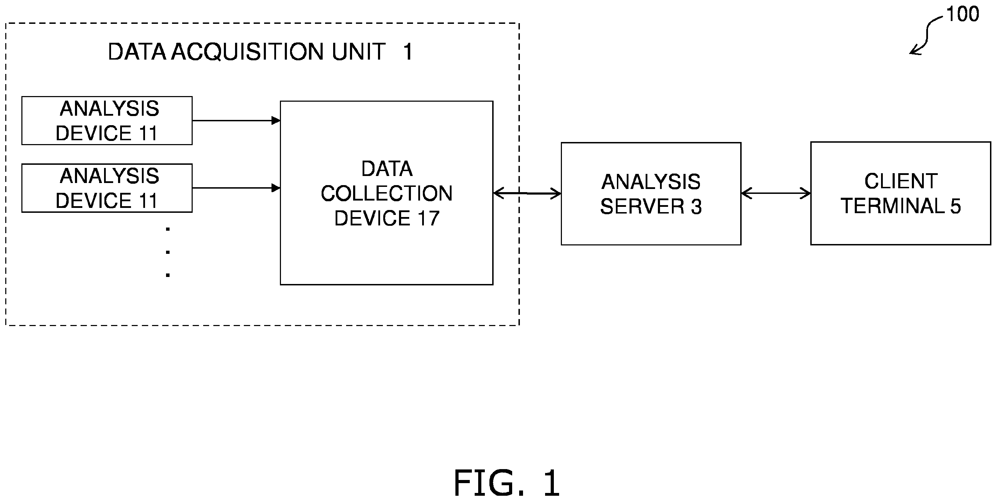

Hereinafter, with reference to Fig. 1, an analysis system 100 is described. Fig. 1 is a diagram illustrating a structure of the analysis system. The analysis system 100 is a system that mainly includes a data acquisition unit 1 and an analysis server 3.

The data acquisition unit 1 includes an analysis device 11. The analysis device 11 collects particulate matter existing at a plurality of places in the predetermined facility at a predetermined period, and calculates data relating to the collected particulate matter at the predetermined period, to calculate chronological data relating to the particulate matter. Note that the data relating to the particulate matter is calculated for each of the plurality of places at which the particulate matter has been collected.

The analysis server 3 is a computer system including a CPU, a storage device (such as a RAM, a ROM, an SSD, and a HDD), various interfaces, and the like. The analysis server 3 is connected to the data acquisition unit 1, and acquires information relating to the environment (environment information) in the predetermined facility, based on the data relating to the particulate matter acquired by the data acquisition unit 1. This time, the knowledge was obtained that the environment in the predetermined facility can be monitored by analyzing the particulate matter existing in the predetermined facility to be analyzed.

The analysis server 3 is connected to a client terminal 5. The client terminal 5 is an information terminal such as a personal computer, a tablet terminal, or a smartphone, which is used by a user. Using the client terminal 5 to access to the analysis server 3, the user can perform browsing or the like of the environment information acquired by the analysis server 3.

Note that Fig. 1 illustrates an example of the analysis system 100 including one data acquisition unit 1, one analysis server 3, and one client terminal 5, but the analysis system 100 may include any numbers of the data acquisition units 1, the analysis servers 3, and the client terminals 5.

The predetermined facility to be analyzed by the above the analysis system 100 is, for example, an indoor factory for producing various products, a clean room, an indoor inspection station for various products (such as vehicles), or the like. For instance, in a facility (factory or clean room) for semiconductor processing, particulate matter of one of gold (Au) and chromium (Cr), which are used for wiring, or an alloy of these metals may be generated from a sputter system or the like, for example. In addition, particulate matter containing iodine (I) may be generated. Such the particulate matter can affect the environment in the indoor factory or the clean room.

In addition, for example, in an indoor inspection station for brake test of vehicles, particulate matter containing titanium (Ti) and components of tires (such as rubber components) can be generated from a vehicle. In addition, for example, in a factory in which electronic equipment is soldered, particulate matter containing components of solder (tin (Sn) and other metals) can be generated. Such the particulate matter can also affect the environment in the indoor inspection station or the factory.

### (2) Data Acquisition Unit

### (2-1) Overall Structure

Hereinafter, with reference to Fig. 1, a specific structure of the data acquisition unit 1 provided to the analysis system 100 is described. The data acquisition unit 1 includes a plurality of the analysis devices 11 and a data collection device 17.

The analysis device 11 collects gas (air) in the predetermined facility at predetermined time points (e.g., every hour), collects particulate matter contained in the gas with a collection filter, and calculates mass concentration of the collected particulate matter and information relating to elements contained in the particulate matter, as data RD relating to the particulate matter. The analysis device 11 calculates the above data RD relating to the particulate matter every time when the particulate matter is collected. Here, "mass concentration" is defined as a mass of the particulate matter contained in the total volume of the collected gas, which is expressed as a value per unit volume of the collected gas (the unit is µg/m³, for example).

Here, the "information relating to elements contained in the particulate matter" means elements contained in the particulate matter and contents of the elements. Further, the "contents of the element" has two types of definitions as follows. In the first definition, the "contents of the element" is defined as how much mass of a target element (or substance) is contained in the total mass of the collected particulate matter, which is expressed as a ratio of the contained mass of the target element (substance) to the total weight of the particulate matter (the unit is mg/mg, for example). In the second definition, the "contents of the element" is defined as element content of a specific element contained in the particulate matter in the total volume of the collected gas, which is expressed as a value per unit volume of the gas (the unit is ng/m³, for example).

In addition, the information relating to elements contained in the particulate matter may include information relating to elements contained in the particulate matter (e.g., element ratio). As the data acquisition unit 1 includes the analysis device 11, the analysis system 100 can perform analysis relating to the particulate matter, based on features extracted from the mass concentration of the particulate matter, and/or the information relating to elements contained in the particulate matter.

Although the particulate matter can vary depending on the source or the like, it contains, for example, gold (Au), chromium (Cr), titanium (Ti), iodine (I), tin (Sn), copper (Cu), iron (Fe), aluminum (Al), silicon (Si), lead (Pb), zinc (Zn), mercury (Hg), vanadium (V), calcium (Ca), potassium (K), arsenic (As), selenium (Se), sulfur (S), an element that causes a flame reaction (such as strontium (Sr)), and the like. The analysis device 11 can acquire information relating to at least these elements and other elements (contained elements, contents of the elements).

A specific structural example of the analysis device 11, which can acquire the mass concentration of the particulate matter and the information relating to elements contained in the particulate matter, will be described later.

The data collection device 17 is a data logger, which acquires the data RD relating to the particulate matter acquired by the plurality of analysis devices 11, and sends the data RD to the analysis server 3. The data collection device 17 determines timing to acquire the data RD relating to the particulate matter from each analysis device, in consideration of time deviation between each analysis device and the data collection device 17. In this way, the data collection device 17 does not fail to acquire the data RD acquired by each analysis device. In addition, the data collection device 17 associates the data RD acquired from each analysis device with time point (time stamp) when the data RD was acquired. When sending the data RD to the analysis server 3, the data collection device 17 also sends the time stamp associated with the data RD to the analysis server 3.

With the above structure, the data acquisition unit 1 can acquire the mass concentration of the particulate matter and the information relating to elements contained in the particulate matter, as the data RD relating to the particulate matter, to provide the same to the analysis server 3. In addition, because the analysis device 11 calculates the data RD every predetermined time when collecting the particulate matter, the data RD is data having its result varying along time. In other words, the data RD is chronological data in which results (such as values) acquired at the time points are chronologically arranged.

In the data acquisition unit 1, an analysis device other than the above analysis device 11 may be connected to the data collection device 17. For instance, the data collection device 17 may be connected to a device for measuring wind direction and speed in the predetermined facility, a device for analyzing gas components (e.g., gas such as hydrocarbon, carbon monoxide (CO), carbon dioxide (CO₂), nitrogen oxides (NOₓ), ozone (O₃), sulfur oxides (SOₓ), hydrogen disulfide (H₂S), and/or volatile organic compounds (VOCs) such as acetone, ethanol, toluene, benzene, and chlorofluorocarbon) in the predetermined facility, a device for measuring temperature, humidity, pressure, or the like in the predetermined facility, a device for measuring flow rate of gas or liquid used in the predetermined facility.

In this way, data of not only the particulate matter existing in the predetermined facility, but also gas components existing in the facility, an air flow in the facility, temperature, humidity, or pressure in the facility, a flow rate of gas or liquid used in the facility, or the like is acquired, and thus more detailed environment information relating to the environment in the predetermined facility can be acquired, based on the data.

### (2-2) Structure of Analysis Device

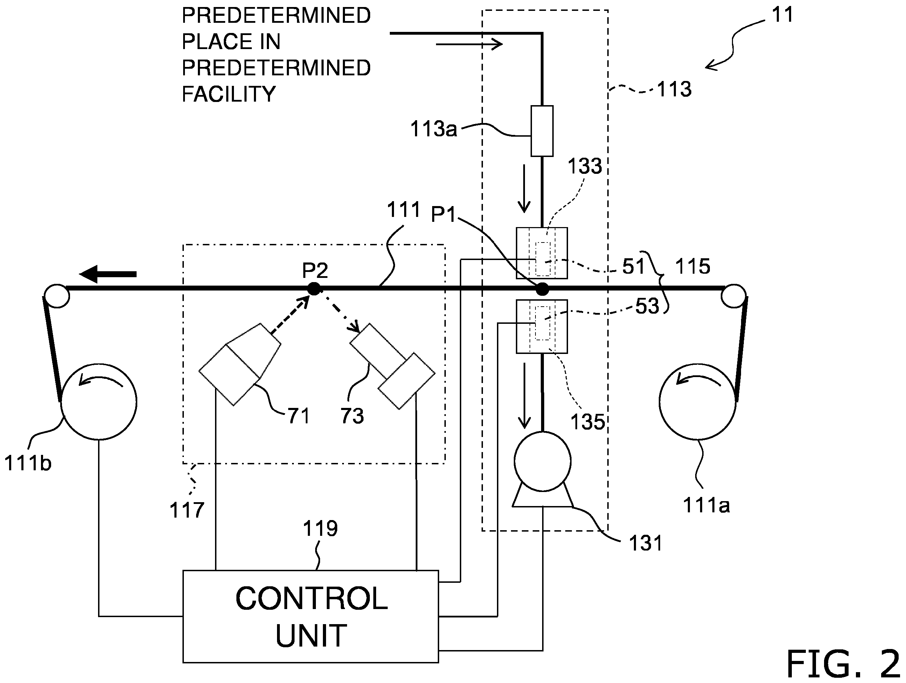

With reference to Fig. 2, a specific structural example of the analysis device 11 is described. Fig. 2 is a diagram illustrating a structural example of the analysis device. The analysis device 11 includes a collection filter 111, a collection unit 113, a first analysis unit 115, a second analysis unit 117, and a control unit 119.

The collection filter 111 is a tape-like member, for example, which includes a reinforcement layer made of nonwoven fabric of a polymer material (such as polyethylene), and a collection layer (also referred to as a collection area) made of porous fluorocarbon resin material having pores capable of collecting the particulate matter, which is formed and laminated on the reinforcement layer. As the collection filter 111, for example, other filter such as a single layer of glass filter or a single layer of fluorocarbon resin material filter can also be used.

In this embodiment, the collection filter 111 can move in the length direction (the direction illustrated by a thick arrow in Fig. 2), when a take-up reel 111b rotates and winds the collection filter 111 fed out from a feed reel 111a.

One end of the collection unit 113 is disposed to correspond to a first position P1 of the collection filter 111 in the length direction. In contrast, the other end of the collection unit 113 is disposed at a predetermined position in the predetermined facility to be analyzed. Specifically, the other end of the collection unit 113 is disposed, for example, at upstream and downstream of the source of the particulate matter or a place where the particulate matter moves with air in the predetermined facility. The part from the other end of the collection unit 113 to a particle size classifier 113a described later is formed of a pipe, for example.

In the case where the part from the other end of the collection unit 113 to the particle size classifier 113a is formed of a pipe, it may be possible to dispose a heater or the like on the side of the particle size classifier 113a, to remove moisture contained in the gas while moving the gas containing the particulate matter to the analysis device 11. It may be possible that the other end of the collection unit 113 is provided with a filter for removing particles that are not the objects to be analyzed.

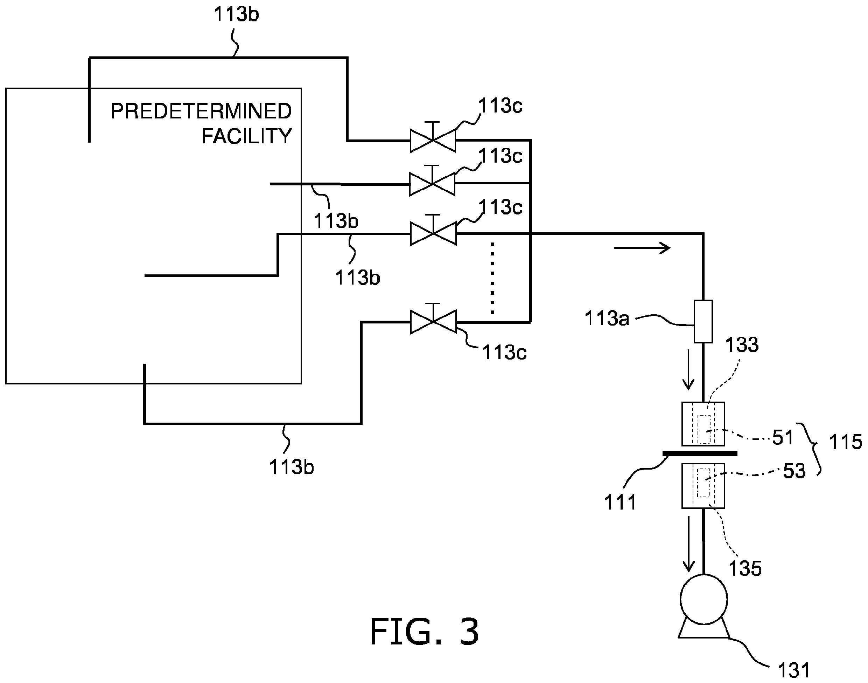

Note that there may be a plurality of the other ends of the collection unit 113. In other words, the single analysis device 11 may be capable of collecting the particulate matter at a plurality of places. Specifically, as illustrated in Fig. 3, by extending each of a plurality of other ends 113b of the collection unit 113 to a desired place in the predetermined facility to be analyzed, via a valve 113c, it is possible to collect the particulate matter from any one of the plurality of places. The place from which the particulate matter is collected can be selected by opening any one of a plurality of the valves 113c while closing other valves. Fig. 3 is a diagram illustrating an example of the collection unit having the plurality of other ends. In this case, in order to prevent the particulate matter at different places from mixing with each other, it may be possible to supply clean gas to the part from the other end of the collection unit 113 to the particle size classifier 113a to clean the part, each time when collecting the particulate matter.

The collection unit 113 blows the air, which is sucked by suction force of a suction port 135 connected to a suction pump 131, for example, through the above pipe or the like, from a discharge port 133 to the collection area at the first position P1 of the collection filter 111, so that the particulate matter contained in the air is collected by the collection area.

The collection unit 113 includes the particle size classifier 113a disposed between the other end of the collection unit 113 and the discharge port 133. The particle size classifier 113a classifies the particulate matter sucked by the collection unit 113, by its particle size. The particle size classifier 113a is, for example, an impactor type particle size classifier. The particle size classifier 113a classifies the particulate matter having a particle size of approximately 2.5 µm or approximately 10 µm, for example, and causes the collection filter 111 to collect the particulate matter. In this way, the data RD relating to the particulate matter having a specific particle size can be acquired. As a result, the environment in the predetermined facility can be monitored in detail.

The first analysis unit 115 measures collected amount of the particulate matter collected by the collection filter 111. Specifically, the first analysis unit 115 includes a β-ray source 51 and a β-ray detector 53. The β-ray source 51 is disposed at the discharge port 133 of the collection unit 113, to emit beta rays to the collection area of the collection filter 111 disposed at the first position P1. The β-ray source 51 is a β-ray source using carbon-14 (14C), for example.

The β-ray detector 53 is disposed at the suction port 135 of the collection unit 113, to face the β-ray source 51, and measures intensity of the beta rays after passing through the particulate matter collected by the collection area of the first position P1. The β-ray detector 53 is a photo multiplier equipped with a scintillator, for example. The collected amount (mass concentration) of the particulate matter is calculated based on the intensity of the beta rays measured by the β-ray detector 53.

The second analysis unit 117 is disposed to correspond to a second position P2 in the length direction of the collection filter 111, and measures data relating to fluorescent X-rays generated from the particulate matter existing in the second position P2. Specifically, the second analysis unit 117 includes an x-ray source 71 and a detector 73.

The x-ray source 71 emits X-rays to the particulate matter existing at the second position P2. The x-ray source 71 is a device that generates X-rays by irradiating a metal such as palladium with an electron beam, for example. The detector 73 detects fluorescent X-rays generated from the particulate matter. The detector 73 is a silicon semiconductor detector or a silicon drift detector, for example.

The control unit 119 calculates data for calculating the mass concentration of the particulate matter using the first analysis unit 115 disposed at the first position P1. In addition, the control unit 119 controls the take-up reel 111b to move the collection filter 111, in order to calculate an element analysis result using the second analysis unit 117 disposed at the second position P2. Specifically, every time when the collection of the particulate matter by the collection unit 113 is finished and measurement of the collected amount is completed (every predetermined period), the control unit 119 moves the collection area of the collection filter 111 (the area where the particulate matter is collected), from the first position P1 where the first analysis unit 115 is disposed to the second position P2 where the second analysis unit 117 is disposed.

After the collection area reaches the second position P2, the control unit 119 controls the x-ray source 71 to emit X-rays to the second position P2, and acquires fluorescent X-rays, which is generated from the particulate matter on the collection area due to the irradiation with the X-rays, as data for element analysis.

The control unit 119 calculates the mass concentration of the particulate matter as the data RD relating to the particulate matter, based on the intensity of the beta rays measured by the first analysis unit 115. In addition, the control unit 119 acquires the fluorescent X-ray data (e.g., fluorescent X-ray spectrum) acquired by the second analysis unit 117, and based on the fluorescent X-ray data, it calculates the elements contained in the particulate matter and the contents thereof, as the data RD relating to the particulate matter. The calculated data RD described above is sent to the data collection device 17.

### (3) Functional Block Structure of Analysis Server

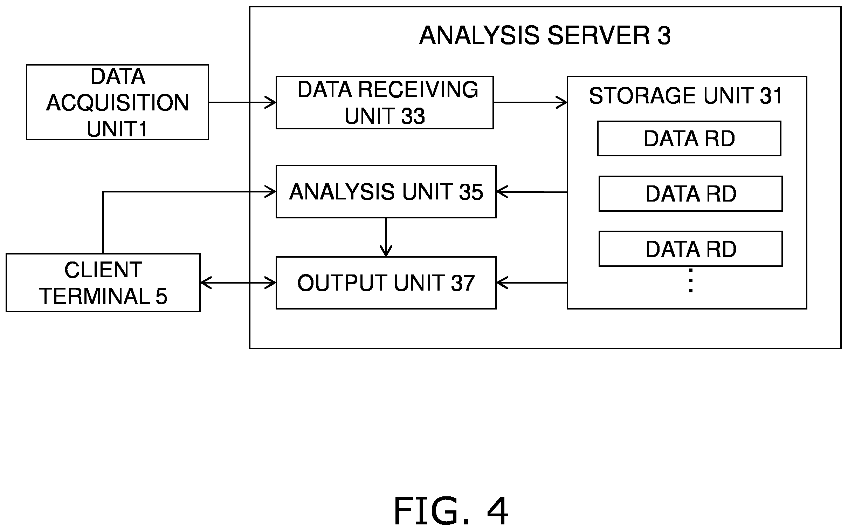

Hereinafter, with reference to Fig. 4, a functional block structure of the analysis server 3 is described. Fig. 4 is a diagram illustrating the functional block structure of the analysis server. The functional blocks of the analysis server 3 described below may be realized by a program, which is stored in the storage device of the analysis server 3 and can be executed by the analysis server 3. In addition, a part of the functional blocks may be realized by hardware constituting the analysis server 3. The analysis server 3 includes a storage unit 31, a data receiving unit 33, an analysis unit 35, and an output unit 37, as the functional blocks.

The storage unit 31 stores various data, programs, setting values, and the like, which are used by the analysis server 3. Specifically, the storage unit 31 stores the data RD acquired by the data acquisition unit 1. Including the storage unit 31, the analysis server 3 functions as a database of the data RD.

The data receiving unit 33 receives the data RD from the data collection device 17 of the data acquisition unit 1, and stores the same in the storage unit 31. The data receiving unit 33 receives the data RD stored in the data collection device 17 at predetermined timing. Note that the data receiving unit 33 may receive the data RD via the data collection device 17 just after each analysis device 11 outputs the data RD (i.e., the data collection device 17 does not accumulate the data RD), or may receive the data RD at timing when some amount of the data RD are accumulated in the data collection device 17.

The analysis unit 35 performs predetermined feature extraction processing of the data RD stored in the storage unit 31 as an input, to extract features contained in the data RD. Specifically, the analysis unit 35 performs a function of searching a peak value (an instantaneous value) contained in the data RD (a peak search function), a function of calculating the average value or median of values contained in the data RD (an average value calculation function), or a function of automatically calculating correlation of a plurality of data RD (an automatic correlation calculation function), and acquires a result calculated by the function as the environment information relating to the environment in the predetermined facility.

The analysis unit 35 may perform the peak search function, the average value calculation function, or the automatic correlation calculation function in accordance with a command from the client terminal 5, or may automatically perform the function at predetermined timing. In addition, when the automatic correlation calculation function is performed, it may be possible that the user can select the plurality of data RD for which the correlation is to be calculated, by using the client terminal 5, or that the analysis unit 35 automatically extracts the plurality of data RD for which the correlation is to be calculated.

The output unit 37 outputs the environment information acquired by the analysis unit 35 to the client terminal 5. When the analysis unit 35 performs the peak search function, the output unit 37 causes the client terminal 5 to display the time point when the peak value occurred. In addition, when the average value calculation function is performed, the output unit 37 causes the client terminal 5 to display information relating to the calculated average value or median. Note that when the occurrence time point of the peak value and/or the average value is output, it may be associated with a link relating to the data RD in which the peak value occurred and/or the data RD for which the average value or median calculated. When this link is selected, the output unit 37 may cause the client terminal 5 to display data values of the corresponding data RD and/or a graph of the corresponding data RD.

When the analysis unit 35 performed the automatic correlation calculation function, the output unit 37 causes the client terminal 5 to display a scatter diagram of the plurality of data RD used for correlation calculation. For instance, concerning a combination of the data RD (element data) relating to contents of two elements for which the correlation relationship was calculated, the output unit 37 can generate and display the scatter diagram of values (contents) included in the element data.

Other than that, the output unit 37 can display the scatter diagram for the plurality of data RD that the user specified using the client terminal 5. For instance, concerning the plurality of elements that the user specified using the client terminal 5, the output unit 37 can generate and display the scatter diagram of contents for a combination of two elements among the plurality of elements. In addition, for example, if the user selects three elements, the client terminal 5 can display the scatter diagram of contents of two elements, for each of combinations (three combinations) of the two elements selected further from the three elements. In this way, the user can visually check the correlation relationship for the plurality of data RD relating to the particulate matter.

Other than that, when the analysis unit 35 performs the automatic correlation calculation function, so that the scatter diagrams for the plurality of combinations of the data RD are output to the client terminal 5, the output unit 37 may display the scatter diagram of the combination of the data RD having large correlation in highlight.

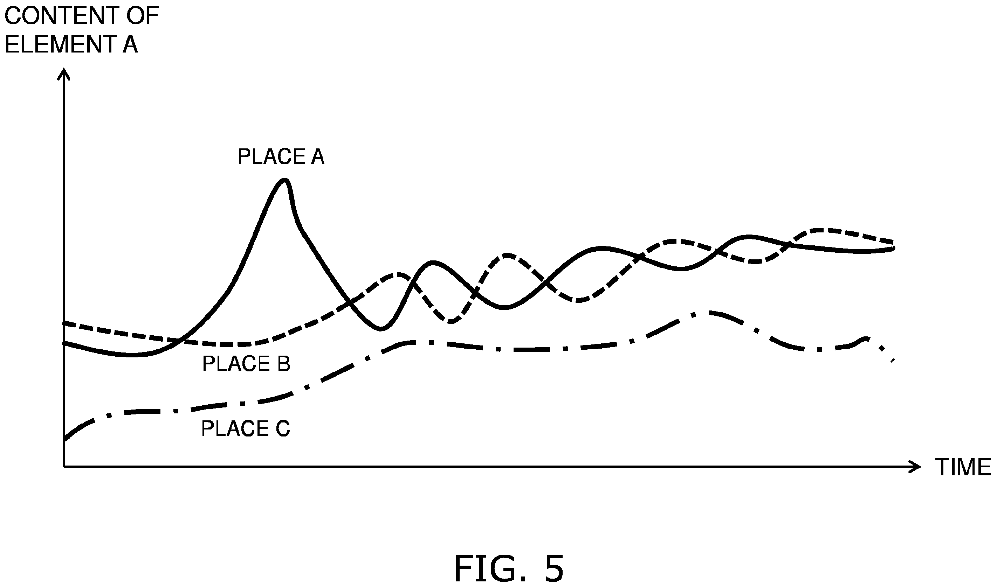

In addition, when the analysis device 11 collected the particulate matter at the plurality of places in the predetermined facility, so that the data relating to the particulate matter at each of the plurality of places was calculated, the output unit 37 may cause the client terminal 5 to simultaneously display graphs of the data RD relating to the particulate matter at the plurality of places. For instance, if data RD relating to the particulate matter were calculated for a place A, a place B, and a place C in the predetermined facility, the three graphs as illustrated in Fig. 5 can be simultaneously displayed. Fig. 5 is a diagram illustrating an example in which graphs of the plurality of data are simultaneously displayed.

In the graphs illustrated in Fig. 5, for example, it is possible to visually check a tendency that a specific peak value occurs in the place A at a specific time point, and then a corresponding peak value occurs in the place B after the specific time point, concerning the data RD relating to content of the element A. From this, it can be estimated that the particulate matter containing the element A has flown from the place A to the place B, and that its source is near the place A. In this way, by simultaneously displaying the graphs of the data RD relating to the particulate matter at the plurality of places, chronological changes of the particulate matter at the plurality of places in the predetermined facility can be visually checked. In addition, based on the chronological changes of the particulate matter, changes of the environment in the predetermined facility can be chronologically monitored in more detail.

### (4) Data Analysis Operation in Analysis System

### (4-1) Peak Search Operation

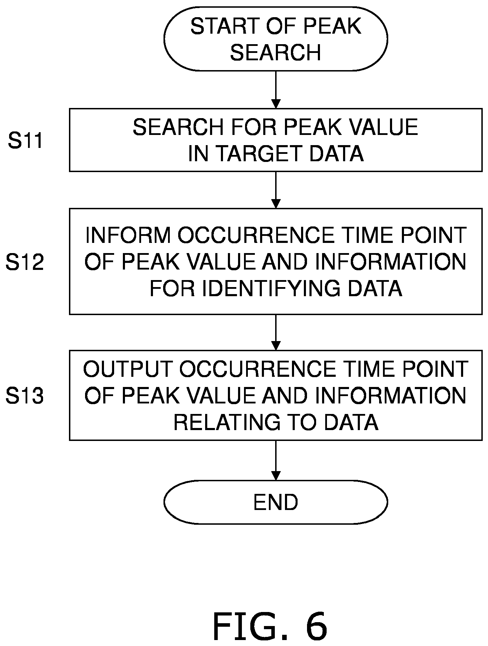

Hereinafter, an analysis operation of the data RD in the analysis system 100 is described. First, with reference to Fig. 6, an operation of the peak search function (a peak search operation) is described. Fig. 6 is a flowchart illustrating the peak search operation. The peak search operation illustrated in the flowchart of Fig. 6 is executed by the analysis server 3.

Note that the peak search operation may be executed on all the data RD (including values varying along time) stored in the storage unit 31, or may be executed on the plurality of data RD that are specified in advance. For instance, it can be executed on the data RD (e.g., the data RD relating to content of a specific element) acquired during the period while a specific wind direction is shown. By the peak search of the data RD acquired during a specific wind direction, a state of the particulate matter from a specific source can be monitored, for example.

For instance, if the peak search is performed on the data RD showing content of an element contained in the particulate matter, when a specific wind direction is observed, it is possible to extract the data RD whose peak value of content of the element contained in the particulate matter is larger than a predetermined threshold value. In this way, it is possible to extract data showing abnormality of the particulate matter that has flown from a specific direction, i.e., the particulate matter that has flown from a specific source.

First, in Step S11, the analysis unit 35 searches for a peak value contained in the data RD as a target of the peak search. For instance, if the data RD is data of content of an element contained in the particulate matter, a peak value of the content is searched. For instance, the analysis unit 35 searches values contained in the data RD one by one. If the currently searched value is larger than values before and after the same, the currently searched value is determined as a sub peak value, and the largest sub peak value among all the sub peak values included in the data RD is determined as the peak value.

Next, for the peak value found by execution of Step S11, the analysis unit 35 informs the output unit 37 of the time point when the peak value occurred, and information for identifying the data RD including the peak value (Step S12).

The output unit 37, which has received the above time point and information for identifying the data RD, causes the client terminal 5 to display the time point when the peak value occurred, in Step S13. In addition, based on the information for identifying the data RD determined in Step S12, the output unit 37 causes the client terminal 5 to display a graph indicating a chronological change of the value in the data RD.

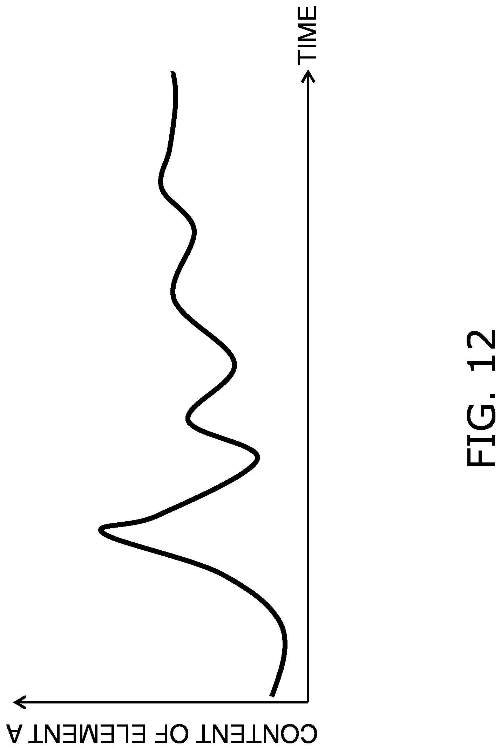

By the above peak search operation, the analysis server 3 can automatically, efficiently, and accurately extract the peak value included in the data RD. In addition, by causing the client terminal 5 to display the graph of the data RD including the peak value as illustrated in Fig. 12, the user can visually check a chronological variation of the data RD including an abnormality, for example. Fig. 12 is a diagram illustrating an example of a data graph display.

### (4-2) Automatic Correlation Calculation Operation

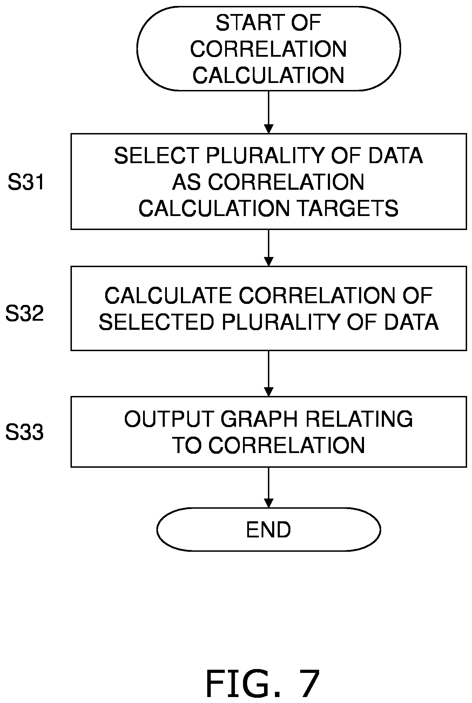

With reference to Fig. 7, an operation of an automatic correlation calculation function is described. Fig. 7 is a flowchart illustrating an automatic correlation calculation operation. The automatic correlation calculation operation illustrated in the flowchart of Fig. 7 is performed by the analysis server 3.

First, in Step S31, the analysis unit 35 selects a plurality of the data RD (two data RD) for which correlation is to be calculated. For instance, before execution of Step S31, the user selects the plurality of data RD for which correlation is to be checked (a plurality of data items for which correlation is to be checked), by using the client terminal 5. After that, in Step S31, the analysis unit 35 that has received the selection from the user selects two data RD for which correlation is to be calculated, from the plurality of data RD selected by the user. Alternatively, the analysis unit 35 may automatically select two data RD for which correlation is to be calculated, from all the data RD stored in the storage unit 31.

Next, in Step S32, the analysis unit 35 calculates correlation of the two data RD selected by execution of Step S31. Specifically, the analysis unit 35 uses values included in one of the two data RD as first elements (x) and values included in the other as second elements (y), to calculate a coefficient of determination indicating a degree of correlation of the two data RD (a square of a correlation coefficient).

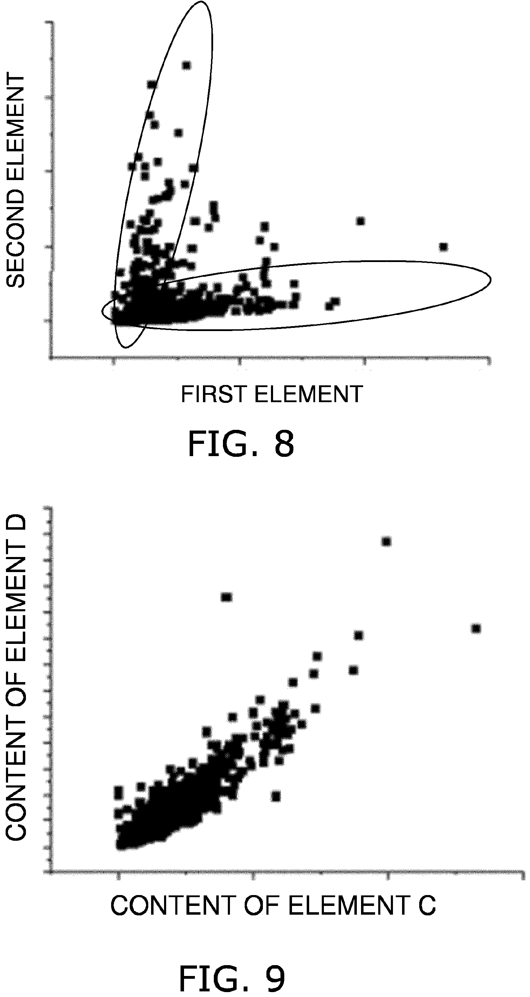

Because the data acquisition unit 1 acquires a value of the data RD every predetermined time, the data RD includes a plurality of values varying along time. If the data RD includes a large number of values, i.e., if the data RD has been acquired as a result of measurement over a long period, a plurality of large correlations may be observed between the two data RD as illustrated in Fig. 8. In the example illustrated in Fig. 8, two large correlations are observed in areas, each enclosed by an ellipse. Note that the coefficient of determination calculated for values of the data RD including a plurality of large correlations has a tendency to be small. In other words, the coefficient of determination calculated from the data RD of a long period may not be able to show that there are a plurality of large correlations. Fig. 8 is a diagram illustrating an example of the scatter diagram in a case where a plurality of correlations are observed between two data.

If a plurality of large correlations are observed between a plurality of data RD, it means that features of the particulate matter collected at a specific place in a predetermined facility have changed during the period of acquiring the data RD, for example. In other words, it means a possibility that the environment at the specific place in the predetermined facility has changed. It is important for analyzing the particulate matter to grasp the timing when features of the particulate matter changed during the period of acquiring the data RD.

Therefore, the analysis unit 35 not only calculates the coefficient of determination using values during the whole period of acquiring the data RD, but also calculates the coefficient of determination using values during a small period after dividing the whole period into a plurality of small periods. For instance, if the data RD for which correlation is to be checked is data acquired for one year, the coefficient of determination is calculated using values of first one month included in the data RD, and then the coefficient of determination is calculated using values of next one month, which is repeated so that total 12 coefficients of determination can be calculated for the data RD acquired for one year.

As described above, by calculating the coefficient of determination for each of the plurality of small periods included in the data RD acquired for a long period, information relating to an event generated in the small period can be acquired. For instance, if the coefficient of determination in a specific small period is small (correlation between the data RD is small), while if the coefficient of determination in another small period is large (correlation between the data RD is large), it can be estimated that a particular event regarding generation of the particulate matter has occurred between these two small periods. In other words, it can be estimated that the environment at the specific place in the predetermined facility has changed between the two small periods.

The above small period may be variable. For instance, it is possible to calculate the coefficient of determination using values in the first one month included in the data RD, and then to calculate the coefficient of determination using values in the first two months included in the data RD. In this way, by enabling to flexibly set the above period, it is possible to identify the period in which the correlation of data changed. For instance, if a large correlation is not observed in the data RD (the coefficient of determination is small) in the first one month, while if a relatively large correlation is observed in the data RD (the coefficient of determination is relatively large) in the first two months, it can be specified that the correlation of the data RD changed at least in the second half of the two months. In other words, it can be specified that the features of the particulate matter changed at least in the second half of the two months.

In addition, it may be possible to further divide the above small period into small intervals, and to calculate the coefficient of determination for each of the small intervals. For instance, it is possible to divide the data RD acquired for one year into data of small periods of one month each, and to further divide the data of the small period of one month into data of small intervals of one week each. In this case, it is possible to acquire the information relating to an event generated in the small interval more finely. Furthermore, the above small interval may be variable similarly to the small period.

As described above, when calculating the correlation of the two data RD (the coefficient of determination), by dividing the data RD into data of short periods to calculate the coefficient of determination, timing or the like when a change of features of the particulate matter occurred can be analyzed more finely, and timing or the like when a specific event occurred in a source or the like of the particulate matter can be finely analyzed. As a result, changes of the environment in the predetermined facility can be analyzed in detail.

If a particularly large coefficient of determination was calculated by the above processing, the analysis unit 35 may inform the output unit 37 of information for identifying the two data RD for which the large coefficient of determination was calculated. In this case, the analysis unit 35 may inform the output unit 37 of information relating to the period in which the coefficient of determination is large, together with the information for identifying the two data RD. Furthermore, the output unit 37 may cause the client terminal 5 to display the information as the environment information.

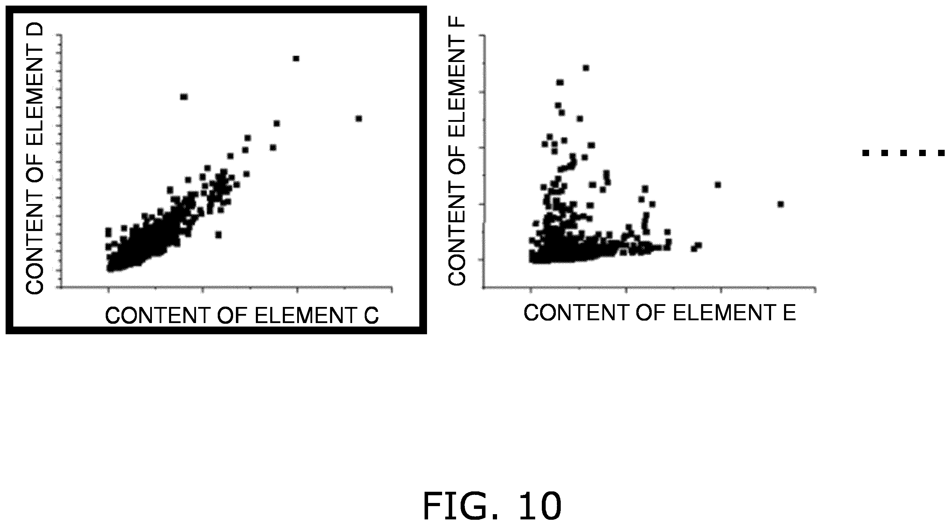

Next, the output unit 37 causes the client terminal 5 to display a scatter diagram of the combination of the data RD for which the correlation was calculated. In this case, as illustrated in Fig. 9, the output unit 37 may generate a scatter diagram of values during the informed period of the two data RD in which the correlation was large, and output the same to the client terminal 5. Alternatively, as illustrated in Fig. 10, it may be possible to display a plurality of scatter diagrams for all the combinations of two data RD, and to display a scatter diagram having a large correlation (coefficient of determination) in highlight by enclosing it with a frame, for example. Fig. 9 is a diagram illustrating an example when displaying only the scatter diagram having a large correlation. Fig. 10 is a diagram illustrating an example when the scatter diagram having a large correlation is displayed in highlight.

In addition, the output unit 37 can display a graph of the chronological changes of the plurality of data RD having a large correlation, based on the information for identifying the two data RD and the period in which the correlation of the two data RD is large, informed by the analysis unit 35. Which one of the scatter diagram and the graph of the chronological changes should be displayed, or that both of them should be displayed can be arbitrarily determined.

By the above automatic correlation calculation operation, the analysis server 3 can automatically, efficiently, and accurately extract the features relating to the correlation of the plurality of data RD. For instance, if the plurality of element contents have a large correlation, it can be estimated that the particulate matter containing the plurality of element at the same content (element ratio) is measured in the period in which the correlation is observed.

In addition, by displaying the scatter diagram of the two data RD having a large correlation, a magnitude of the correlation of the two data RD can be visually checked. In addition, by displaying a plurality of scatter diagrams regardless of the magnitudes of the correlation, and by displaying the scatter diagram having a large correlation in highlight among the displayed plurality of scatter diagrams, it is possible to visually check which one of the data RD has a large correlation among all the data RD.

Furthermore, by displaying a graph of a temporal change of the correlation of the plurality of data RD having a large correlation, it is possible to visually check how the correlation of the plurality of data RD changes chronologically.

For instance, if the data RD is data indicating contents of elements contained in the particulate matter, it is possible to visually check how the correlation of the plurality of element contents changes chronologically. If the correlation between the plurality of element contents is large, it means that the particulate matter contains a plurality of elements having a large correlation at a constant composition ratio. Elements contained in the particulate matter and composition ratios thereof depend on features of the particulate matter such as a source and/or occurrence conditions or the like of the particulate matter.

Therefore, by displaying a graph of the temporal change of the correlation of the plurality of element contents having a large correlation, the user can visually check the temporal change of the correlation, for example, and can estimate a change of features of the particulate matter, i.e., when and from which source the particulate matter occurred, and/or when and how the occurrence conditions of the particulate matter changed.

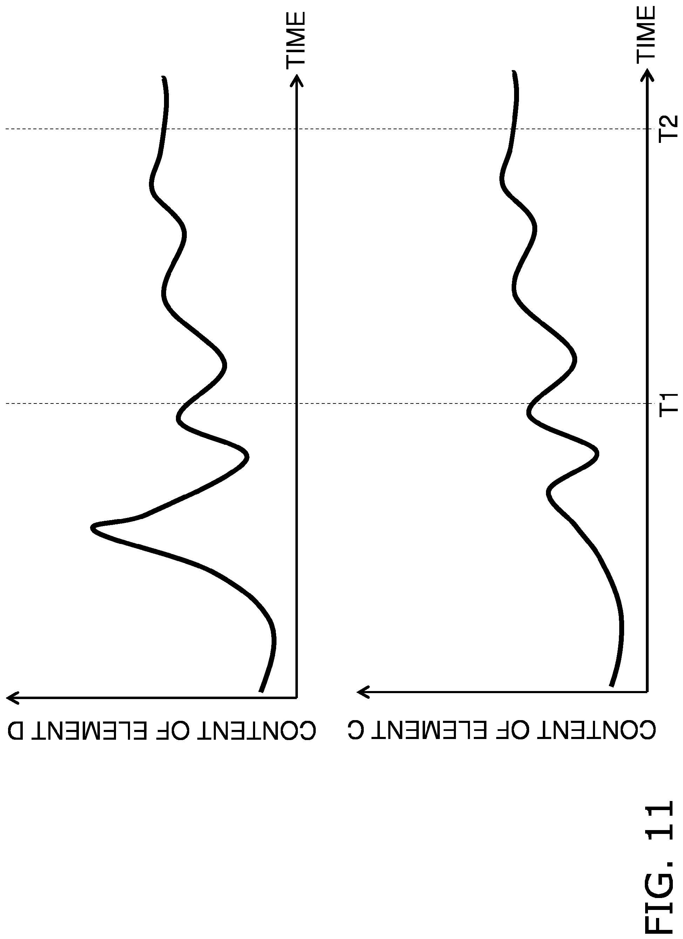

Note that, if this scatter diagram is specified during displaying the scatter diagram of the two data RD having a large correlation, the output unit 37 may display the graphs of the chronological changes of values of the two data RD for which this scatter diagram was generated, as illustrated in Fig. 11. Fig. 11 is a diagram illustrating an example of a state where the chronological changes of the two data values having a large correlation are displayed in graphs. Fig. 11 illustrates an example where the scatter diagram of content of an element C and a content of an element D having a large correlation as illustrated in Fig. 9 is specified, and the chronological change of content of the element C and the chronological change of content of the element D are parallelly displayed in graphs.

Only the scatter diagram illustrated in Fig. 9 cannot clarify the period in which the correlation of the two data RD is large, but by displaying the graph of the chronological change as illustrated in Fig. 11, it is possible to estimate the period in which the correlation is large. In the example illustrated in Fig. 11, the content of the element C and the content of the element D have the same increase and decrease tendency during the period between time point T1 and time point T2, and it can be estimated that the content of the element C and the content of the element D have a large correlation during this period.

When generating the scatter diagram of the two data RD as illustrated in Fig. 8, the output unit 37 may output to the client terminal 5 the data RD with coloring the points during the period having a high correlation. In this way, even if the two data RD include a plurality of periods having a high correlation, the plurality of correlations of the two data RD having a high correlation can be distinguished by the color of the points of the scatter diagram. For instance, if the two data RD are data relating to the particulate matter generated from the same source, it is possible to visually check that features of the particulate matter (such as composition ratios of elements) have changed over time.

### 3. Other Embodiments

Although the plurality of embodiments of the present invention are described above, the present invention is not limited to the above embodiments but can be variously modified within the scope of the invention without deviating from the spirit thereof. In particular, the plurality of embodiments and variations can be arbitrarily combined as necessary.
(A) The orders and/or contents of the steps of the flowchart illustrated in Fig. 6 or 7 can be modified within the scope of the invention without deviating from the spirit thereof.
(B) The above peak search function or average value calculation function may be provided to the data collection device 17 of the data acquisition unit 1. If the data RD stored in the data collection device 17 includes a peak value of a first threshold value or more, and/or an average value of a second threshold value or more, the data collection device 17 having the peak search function or the average value calculation function may send the fact and/or a warning externally via electronic mail.
   More specifically, for example, among the data RD stored in the data collection device 17, if there is any data RD in which concentration of a specific component detected in a specific wind direction exceeds a certain value for a certain period of time, the data collection device 17 can issue warning mail.
(C) When calculating the correlation using element ratios of elements contained in the particulate matter in the automatic correlation calculation operation, it may be possible to perform the calculation of the correlation (coefficient of determination) after removing data having an extremely small or large element ratio.
(D) In the above first embodiment, the analysis system 100 is a so-called "client-server system" having the analysis server 3, but this is not a limitation. For instance, the data acquisition unit 1 (each analysis device or the data collection device 17 thereof) may have a function of the analysis unit 35 and/or the output unit 37, to constitute an analysis system as so-called "edge computing". In this case, the data acquisition unit 1 may send to the analysis server the features extracted from the data RD, in addition to the data RD.
(E) The analysis unit 35 may realize the feature extraction processing using a machine learning algorithm such as a neural network, for example. Specifically, for example, the data RD having features to be extracted and the features of the data RD are used as training data to generate learned models of the neural network, which can be the analysis unit 35. By inputting the data RD to be analyzed to the analysis unit 35 as a learning model, features included in the data RD can be extracted.
(F) The analysis unit 35 can use any other statistical analytical method such as data mining of the data RD, instead of the statistical analytical method described in the above first embodiment, to extract features included in the data RD.
(G) The analysis unit 35 may set a predetermined threshold value, and perform an algorithm for determining whether or not a data value included in the data RD is more than the threshold value, a number of times, to extract features included in the data RD.

### INDUSTRIAL APPLICABILITY

The present invention can be widely applied to analysis systems for performing analysis relating to the particulate matter.

### REFERENCE SIGNS LIST

100 analysis system
1 data acquisition unit
11 analysis device
111 collection filter
111a feed reel
111b take-up reel
113 collection unit
113a particle size classifier
113b other end
113c valve
115 first analysis unit
117 second analysis unit
119 control unit
131 suction pump
133 discharge port
135 suction port
51 β-ray source
53 β-ray detector
71 x-ray source
73 detector
17 data collection device
3 analysis server
31 storage unit
33 data receiving unit
35 analysis unit
37 output unit
5 client terminal

## Claims

1. An analysis system for performing analysis relating to environment in a predetermined facility, comprising:
a data acquisition unit configured to collect particulate matter existing in the predetermined facility at a predetermined period, to calculate chronological data relating to the collected particulate matter; and
an analysis unit configured to acquire environment information relating to the environment in the predetermined facility, based on the data relating to the particulate matter calculated by the data acquisition unit, wherein
the data relating to the particulate matter includes element data relating to elements contained in the particulate matter.

2. The analysis system according to claim 1, wherein
the data acquisition unit collects the particulate matter at a plurality of places in the predetermined facility, to calculate the data relating to the particulate matter at each of the plurality of places, and
the analysis system further comprises an output unit configured to simultaneously output the data relating to the particulate matter at the plurality of places.

3. The analysis system according to claim 1 or 2, wherein the environment information includes correlation between a plurality of the data relating to the particulate matter.

4. The analysis system according to any one of claims 1 to 3, wherein
the data acquisition unit includes a collection unit configured to collect particulate matter in the predetermined facility, and
the collection unit has a plurality of ends extending to a plurality of places in the predetermined facility to collect the particulate matter from the plurality of places.

5. The analysis system according to any one of claims 1 to 4, further comprising an output unit configured to output a scatter diagram of the plurality of element data.

6. The analysis system according to any one of claims 1 to 5, wherein the data relating to the particulate matter includes data relating to mass concentration of the particulate matter.

7. The analysis system according to any one of claims 1 to 4, wherein the data acquisition unit includes a particle size classifier configured to classify the particulate matter by its particle size.

8. An analysis method for performing analysis relating to the environment in a predetermined facility, the method comprising:
collecting particulate matter existing in the predetermined facility at a predetermined period;
calculating chronological data relating to the collected particulate matter; and
acquiring environment information relating to the environment in the predetermined facility, based on the calculated data relating to the particulate matter, wherein
the data relating to the particulate matter includes element data relating to elements contained in the particulate matter.

9. The analysis method according to claim 8, wherein the environment information includes correlation between a plurality of the data relating to the particulate matter.
